# EUROPEAN PATENT SPECIFICATION

(11) **EP 3 868 412 B1**
(45) Date of publication and mention of the grant of the patent: **28.06.2023**
(21) Application number: 21156661.7
(22) Date of filing: 11.02.2021
(51) Int. Cl.: A61L 9/12, A61L 9/03, B65D 39/00, B65D 41/04

(54) **TOP FOR CONTAINER**
OBERTEILE FÜR BEHÄLTER
PARTIE SUPÉRIEURE POUR RÉCIPIENT

(30) Priority: 13.02.2020 ES 202030228 U
(43) Date of publication of application: 25.08.2021
(73) Proprietor: ZENIT Estudio de Diseño e Innovación S.L., 46010 Valencia (ES)
(72) Inventor: BLASCO FEO, VICENTE, 46010 Valencia (ES)
(74) Representative: Soler Lerma, Santiago

(56) References cited:
- EP-A1- 0 740 941
- GB-A- 2 435 038
- US-A1- 2002 195 415
- US-A1- 2014 124 471
- US-A1- 2018 104 463
- US-B1- 6 375 020

## Description

The invention refers to a top for a container, specifically for a container that incorporates a wick which protrudes from its mouth and is housed in a cavity of the top, comprising a bell-like body where it is possible to distinguish an upper part with a hollow tubular shape, suitable for part of the wick to be housed on its inside, a middle zone which has on its internal face means of anchoring to the container, and a lower part or skirt.

In the inner part of the top are stiffeners like inner ribs and on the outside of the top outer ribs, with the inner stiffeners coincident with outer ribs, these contributing to strengthen the part allowing the thickness of the walls of the top to be reduced, achieving a lower consumption of plastic and consequently a reduction in weight.

The sector of the art to which it belongs is closures for containers.

### Background of the art

Today, refills for volatile substance evaporators are well known, and comprise a container including a wick partially inserted into the container, in contact with the substance to be evaporated, and partially outside the container. The part of the wick that is outside of the container, which we shall call outer wick, will have a source of heat nearby to achieve the evaporation of the substance inside the container that impregnates the wick.

The tops for these containers should have a cavity to house and protect the outer wick from production up to when the refill is put to use.

These tops are single-use and, once this container is opened, the top cannot be reused, so it is of particular interest, both from an environmental and economic point of view, to reduce the amount of plastic used and consequently reduce its weight.

Nonetheless, the reduction of the thickness of the walls of the top may lead to a loss of necessary resistance capacity and generate deformations that might cause damage to the product or problems in the production lines.

Accordingly, for example, a flaw in the skirt may lead to deformations, for example when it is threaded, that render it unusable or with deviations in its insertion.

In the ambit of tops for containers, there is no known top that optimizes the use of plastic as does the one now proposed and with the rigidity and robustness it presents, so the prior arts cited are from other sectors.

Patent ES2646757 refers to a volatile substance device that comprises a top which allows it to close said container externally, with no further use than having attached a rigid band and a regulator that enable the impregnation of a substance which, upon unscrewing the top and partially extracting it, enables the diffusion of the substance.

Patent ES2325858 refers to a liquid air freshener device that has a cover which comprises an upper cylindrical body, a flat-based intermediate crown on which there is defined two large openings from where concordant windows made of a tubular segment extend downwards, which in turn is endowed with an inner threaded segment.

The patent EP0740941 refers to a top as a closure screwed onto a neck and is provided with an annular ridge that is adapted to against the sealing gasket. The closure axially forms a tubular portion that is closed at the upper end and is adapted to accommodate, with a limited interspace, the portion of the wick that protrudes from the neck of the container.

The patent US2018104463 refers to a container having a neck surrounding a dispensing outlet, a ball rotatably mounted and restrained in the neck, a valve seat formed within the neck, an outer wall of the neck having an upper portion and a lower portion with the upper portion of the outer wall having threads formed thereon and a lower portion having a locking mechanism formed thereon, a cap with screw threads formed on an upper portion thereof and a locking member formed on a lower portion of the cap side wall.

The utility model ES1098356U refers to a drum plug as used by cyclists which consists of a protruding spout on the threaded top of the drum, has incorporated to the spout a cannula consisting of a tube running internally to the spout body from its embouchure until it crosses its base continuing independent downwards.

None of the tops described solves the load requirement in a top of the type now proposed.

The preceding patents and utility models differ to the present invention since they have no ribs for the effect of minimizing the thickness of the top and provide resistance to its base with the consequent effect of limiting the manufacturing material providing in terms of production a resistance of the assembly part that enables the mass manufacturing thereof without adverse effects associated to deformation or deterioration thereof.

### Description of the invention

The invention refers to a top for closing containers, preferably of the type containing substances to evaporate by way of a wick, adopting the top preferably bell-shaped where it is possible to distinguish the following:
A hollow upper part, preferably cylindrical, closed by its upper base and opened by its lower base such that it generates a cavity suitable for housing the wick. This upper part serves equally as handle.

A middle zone opened by its upper and lower bases, its upper base being connected with the upper part and its lower base with the skirt. On its internal face, this middle zone presents anchoring or fastening elements, for example threads or clipping tabs. We will call the anchoring zone the zone where these elements are disposed. These anchoring elements are normally complementary to those in the container.

A flared skirt, opened by its upper and lower bases, connected through its upper base with the middle zone, its lower base being open to the outside of the top. At its lower base, the skirt has a cornice or stiffening ring.

In the inner part of the top, between the anchoring zone and the skirt, inner ribs like stiffeners are disposed.

The outer surface presents outer ribs, between the middle zone and the skirt, which contribute to give rigidity.

In a possible variation the top may present a groove which can visually be perceived as an extension of the outer ribs, although the ribs have greater depth and robustness than the groove.

In another possible variation the outer surface may present any type of surface, for example rough.

In a possible variation, the upper part of the top presents a greater diameter than the diameter of the middle zone and this in turn small than that of the skirt, generating a bell shape.

Preferably, the base of the upper part has on the inside an extension like an O-ring so that when the top is anchored to the container, close tightly by tightening on a plug disposed in the mouth of the container and which embraces the wick. The inside of the middle zone is partially occupied by the anchoring zone that incorporates the anchoring elements of the top into the container which, in a possible example, will be threaded elements.

On its internal face, the top incorporates inner ribs that run from the anchoring zone to the skirt.

These inner ribs, which we shall call stiffeners, are disposed radially downwards and stiffen the top. The stiffeners are separated from each other and preferably with their larger edge giving the inner cavity of the top.

In a possible embodiment these stiffeners may be shaped like an obtuse triangle.

At least a part of the inner stiffeners can be coincident with outer ribs and in the embodiment all the stiffeners are coincident with outer ribs.

We will say they are coincident when the inner stiffener and the outer rib are aligned longitudinally, that is, that they share at least part of their longitudinal path with the stiffener running inside the top and the outer rib on the outer surface thereof.

In order to minimize the use of manufacturing material, the design of the top has these inner stiffeners and their correlated outer ribs which provide resistance capacity to the part and prevent the production line from deforming the top causing errors in the adjustments, damage to the product or incidences on the production line which slow down or even stop it.

The skirt comprises a stiffening cornice that complements the part and also contributes to strengthen it preventing perimeter deformations.

This stiffening cornice is disposed on the lower perimeter of the skirt coincident with the larger diameter thereof.

At times these containers are joined to other identical or similar ones through a yoke that embraces them by the neck. In this way, refills can be made, for example, for air fresheners of more than one fragrance.

For improved understanding of the foregoing, the accompanying drawings below are illustrative and not limitative of same.

### Description of the drawings

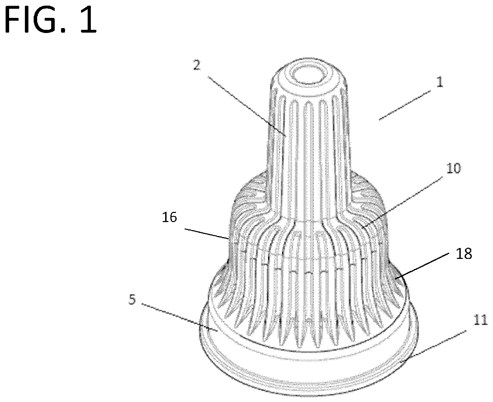

FIGURE 1 is a perspective view of the top (1) showing the upper part (2), the middle zone (16), the skirt (5) and the stiffening cornice (11). In the outside part of the top, the groove (10) and the outer ribs (18) are seen. In this figure the groove (10) appears visually as an extension of the ribs (18), and these outer ribs (18) are deeper than the groove.

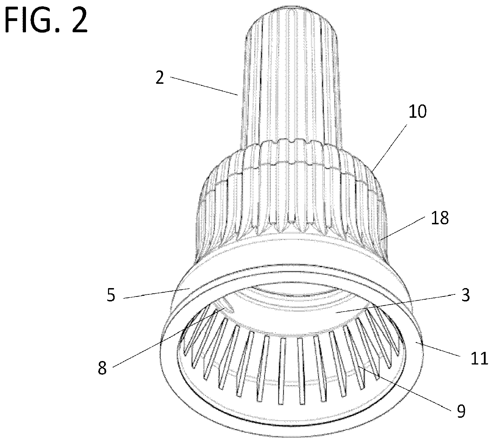

FIGURE 2 displays the top in a lower perspective and shows the upper part (2), the anchoring zone (3) in this case threaded, the thread ring (8), the inner stiffeners (9), the groove (10), the outer ribs (18) the skirt (5) and the cornice (11).

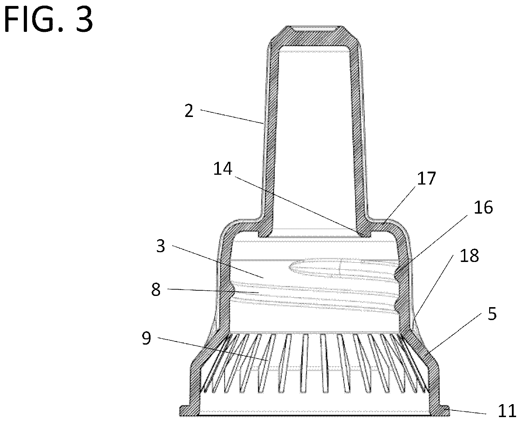

FIGURE 3 shows the side section of the top and the extension can be seen as an O-ring (14) suitable for closing by tightening on the plug inserted into the mouth of the container, not shown here. The threading zone (3) is noted, with the thread ring (8), the inner stiffeners (9), the upper part (2), the skirt (5) and the cornice (11). Moreover, there is also a transition zone (17) that saves the difference in diameter between the upper part (2), the middle zone (16) and the outer ribs (18) that run along the outer surface of the top.

It can be seen that the stiffeners (9) are coincident with the outer ribs (18).

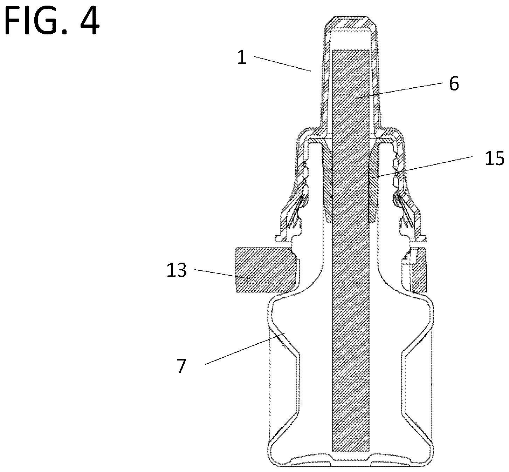

FIGURE 4 shows a sectional view of the mounted assembly with the top (1), the wick (6), the plug (15), the yoke (13) and the container (7) being appreciated.

### Description of a mode of execution of the invention

Below is a description of a mode of execution of the invention which is not the only one, being illustrative rather than limitative.

The invention refers to a top (1) for a container preferably of volatile substances to evaporate by way of an impregnated wick, although the invention may be applied to other types of tops. The top comprises:
An upper part (2) of tubular section presents a smaller diameter than the rest of the top. This section is hollow and is closed by its upper base.

A middle zone (16) that inside has the anchoring zone (3), in this case threaded. This middle zone, with open bases, has a larger diameter than the upper part both being bodies joined by a transition zone (17) that saves the difference in diameters between both. The upper part (2) and the middle zone are connected.

A truncated cone, open-base skirt (5) whose upper base connects with the middle zone (16) and whose open lower base incorporates a stiffening cornice (11). Inner stiffeners (9) disposed between the anchoring zone (3) and the cornice (11). These stiffeners comprise ribs that protrude from the internal face of the top. Outer ribs (18).

The hollow upper part (2) has an inner cavity suitable for housing the part of the wick (6) that protrudes from the container (7) which seals the top. At the lower base of this cavity is an extension like an O-ring (14) suitable for adjusting by tightening on the plug (15) disposed in the mouth of the container (7) for improved sealing thereof.

The anchoring zone (3) comprises a thread ring (8) on its inner face.

Between the anchoring zone (3) and the cornice (11) are inner stiffeners (9)

These stiffeners (9) are disposed radially downwards and against the perimeter of the top, with a separation between them, and are shaped like an obtuse triangle.

The outside of the top (1) has a groove (10) and outer ribs (18). These outer ribs (18) are coincident with the stiffeners (9) such that each inner stiffener (9) is correlative to an outer rib, which enhances the robustness of the top.

The skirt (5) comprises a stiffening cornice (11) endowing the skirt with robustness whereby preventing deformations. This cornice is perimeterly at the base of the skirt, at its largest diameter.

## Claims

1. - A TOP FOR A CONTAINER comprising: a hollow upper part (2) opened through its lower base and having
with a cavity suitable for housing the protruding part of a wick (6) inserted into the container (7), which container is to be closed and fastened by a plug (15);
a middle zone (16) with an anchoring zone (3);
the top **characterized by** also by comprising:
a flared skirt (5), opened at its upper and lower bases and connected through its upper base with the middle zone (16), wherein its lower base is open to the outside of the top; and
a stiffening cornice (11) disposed perimeterly on the skirt (5) at the largest diameter of its lower base;
inner stiffeners (9) disposed radially downwards between the anchoring zone (3) and the cornice (11) in the inner part of the top;
and outer ribs (18), disposed on the outer surface of the top between the middle zone and the skirt,
wherein all of the inner stiffeners (9) are coincident with outer ribs (18).

2. - The TOP FOR CONTAINER according to claim 1 **characterized in that** the inner stiffeners (9) are shaped like an obtuse triangle.

3. - The TOP FOR CONTAINER according to claim 1 **characterized in that** the base of the upper part (2) extends like an O-ring (14) suitable for adjusting by tightening on the plug (15) disposed in the mouth of the container (7).

## Patentansprüche

1. Ein DECKEL FÜR EINEN BEHÄLTER, umfassend: ein hohles Oberteil (2), das an seiner unteren Basis offen ist und einen Hohlraum aufweist, der geeignet ist, den hervorstehenden Teil eines in den Behälter (7) eingeführten Dochtes (6) aufzunehmen, wobei der Behälter durch einen Stopfen (15) verschlossen und befestigt werden soll; einen mittleren Bereich (16) mit einer Verankerungszone (3); wobei der Deckel **dadurch gekennzeichnet ist, dass** er außerdem umfasst: eine aufgeweitete Schürze (5), die an ihrer oberen und unteren Basis offen ist und durch ihre obere Basis mit der mittleren Zone (16) verbunden ist, wobei ihre untere Basis zur Außenseite des Deckels hin offen ist; und ein Versteifungsgesims (11), das am Umfang der Schürze (5) am größten Durchmesser ihrer unteren Basis angeordnet ist; innere Versteifungen (9), die radial nach unten zwischen der Verankerungszone (3) und dem Gesims (11) im inneren Teil des Verdecks angeordnet sind; und äußere Rippen (18), die auf der Außenfläche des Verdecks zwischen der mittleren Zone und der Schürze angeordnet sind, wobei alle inneren Versteifungen (9) mit äußeren Rippen (18) zusammenfallen.

2. Der DECKEL FÜR EINEN BEHÄLTER nach Anspruch 1, **dadurch gekennzeichnet, dass** die inneren Versteifungen (9) die Form eines stumpfen Dreiecks haben.

3. Der DECKEL FÜR EINEN BEHÄLTER nach Anspruch 1, dass sich die Basis des Oberteils (2) wie ein O-Ring (14) erstreckt, der sich durch Festziehen auf den in der Öffnung des Behälters (7) angeordneten Stopfen (15) einstellen lässt.

## Revendications

1. Un COUVERCLE POUR un RÉCIPIENT comprenant: une partie supérieure creuse (2) ouverte à travers sa base inférieure et ayant une cavité appropriée pour recevoir la partie saillante d'une mèche (6) insérée dans le récipient (7), lequel récipient doit être fermé et fixé par un bouchon (15) ; une zone médiane (16) avec une zone d'ancrage (3) ; le couvercle étant **caractérisé en ce qu'**il comprend également : une jupe évasée (5), ouverte à ses bases supérieure et inférieure et reliée à la zone médiane par sa base supérieure (16), dans laquelle sa base inférieure est ouverte vers l'extérieur de la partie supérieure ; et une corniche de raidissement (11) disposée sur le pourtour de la jupe (5) au plus grand diamètre de sa base inférieure ; des raidisseurs intérieurs (9) disposés radialement vers le bas entre la zone d'ancrage (3) et la corniche (11) dans la partie intérieure de la partie supérieure ; et des nervures extérieures (18), disposées sur la surface extérieure de la partie supérieure entre la zone médiane et la jupe, dans laquelle tous les raidisseurs intérieurs (9) coïncident avec des nervures extérieures (18).

2. Le COUVERCLE POUR RÉCIPIENT selon la revendication 1, **caractérisé en ce que** les raidisseurs intérieurs (9) ont la forme d'un triangle obtus.

3. Le COUVERCLE POUR RÉCIPIENT selon la revendication 1, **caractérisé en ce que** la base de la partie supérieure (2) s'étend comme un joint torique (14) approprié pour être ajusté par serrage sur le bouchon (15) disposé dans l'embouchure du récipient (7).
